# EUROPÄISCHE PATENTSCHRIFT

(11) **EP 1 372 737 B1**
(45) Veröffentlichungstag und Bekanntmachung des Hinweises auf die Patenterteilung: **02.08.2006**
(21) Anmeldenummer: 01990310.3
(22) Anmeldetag: 20.12.2001
(51) Int. Cl.: A61K 49/00, A61K 49/04

(54) **ZUBEREITUNG FÜR RESTENOSEPROPHYLAXE**
PREPARATION FOR THE PROPHYLAXIS OF RESTENOSIS
PREPARATION DE PREVENTION DE LA RESTENOSE

(30) Priorität: 26.03.2001 DE 10115740
(43) Veröffentlichungstag der Anmeldung: 02.01.2004
(60) Teilanmeldung: 06001040.2
(73) Patentinhaber: Speck, Ulrich, D-13465 Berlin (DE)
(72) Erfinder: SPECK, Ulrich, 13465 Berlin (DE); SCHELLER, Bruno, 66440 Blieskastel (DE)
(74) Vertreter: Weiss, Wolfgang
(86) Internationale Anmeldenummer: PCT/DE2001/004782
(87) Internationale Veröffentlichungsnummer: WO 2002/076509

(56) Entgegenhaltungen:
- WO-A-94/07484
- DATABASE EMBASE [Online] ELSEVIER SCIENCE PUBLISHERS, AMSTERDAM, NL; JACKSON D.M.A. ET AL: "Current usage of contrast agents, anticoagulant and antiplatelet drugs in angiography and angioplasty in the UK." retrieved from STN Database accession no. 95327068 XP002226116 & CLINICAL RADIOLOGY, (1995) 50/10 (699-704). ,

## Beschreibung

Die Erfindung betrifft eine Zubereitung zur Restenoseprophylaxe sowie deren Aufbringung auf Angiographiekatheter.

Blutgefäßverengungen sind eine wichtige Ursache von Morbidität und Mortalität. Lokale Verengungen oder Verschlüsse von größeren Gefäßen bis minimal ca. 2 mm Durchmesser lassen sich in vielen Fällen mit Hilfe von aufdehnbaren Ballonkathetern wieder auf ihr ursprüngliches Lumen aufweiten. Dabei werden sehr hohe Drucke aufgewendet, die zu Einrissen der verdickten Gefäßwände, deren Quetschung und Verdrängung in das umgebende Gewebe führen können. Bei einem Teil derartiger Eingriffe werden röhrenförmige, perforierte Metallstützen ("Stents") implantiert, um die Gefäße offen zu halten. In vielen Fällen reagieren die derart behandelten Gefäßwände binnen weniger Wochen und Monate mit einem verstärkten Dickenwachstum, das einer Narbenbildung ähnelt. Dadurch und durch die fortschreitende Arteriosklerose kann es in relativ kurzer Zeit zu einer erneuten Gefäßverengung ("Restenose") kommen. Die Restenose ist ein gravierendes medizinisches Problem und verursacht hohe Kosten.

Eine erwiesenermaßen klinisch wirksame Methode, der Restenose vorzubeugen, ist die Bestrahlung der betroffenen Gefäßwandbezirke unmittelbar nach dem Eingriff mit hohen Dosen Röntgenstrahlung (extrakorporale Quellen oder intraluminal plazierte Radioisotope).

Wesentlicher Nachteil der Bestrahlung ist in den notwendigen Vorsichtsmaßnahmen beim Umgang mit therapeutischen Strahlendosen zu sehen. Zahlreiche andere Verfahren zur Verhinderung der vorzeitigen Restenose sind experimentell und klinisch erprobt worden, bisher jedoch ohne entscheidenden Erfolg [Bult H. Restenosis: A challenge for pharmacology. Tips 21 274-279, 2000]. Gute Ergebnisse sind bisher nur mit Arzneimittel-freisetzenden Stents erzielt worden. Für die Wirksamkeit dieser Methode ist die Implantation von Stents unabdingbar, so dass die Prophylaxe der Restenose nach alleiniger Gefäßerweiterung ohne Stentimplantation unmöglich ist.

Eine Hemmung der Mitose, der reaktiven Gefäßwandverdickung und der Restenose ist für eine Vielzahl von Arzneistoffen beschrieben: Wichtige Wirkprinzipien sind Plättchenaggregationshemmung, Enzymhemmung, Mitosehemmung, Zytostatika und Kortikoide. In vitro und z.T. auch tierexperimentell wurden günstige Ergebnisse erzielt, die sich bisher klinisch nicht bestätigen ließen. Als Erklärung wird vor allem angeführt, daß in den betroffenen Abschnitten der Gefäßwände keine ausreichenden Wirkstoffkonzentrationen erreicht werden. Dies gilt besonders für die orale und intravenöse Verabreichung, bei denen Nebenwirkungen eine höhere Dosierung verhindern. Als Alternative wurde die Verabreichung über spezielle Katheter versucht, die entweder die Arzneimittellösung durch die Poren eines eng anliegenden Ballons direkt in die Gefäßwand pressen oder den Zu- und Abfluß in einem Gefäßabschnitt blockieren und die Gefäßwand für einige Zeit der Arzneistofflösung aussetzen [Herdeg,C., M. Oberhoff, D.I. Siegel-Axel, A. Baumbach, A. Blattner, A. Küttner, S. Schröder, K.R. Karsch: Paclitaxel: Ein Chemotherapeutikum zur Restenoseprophylaxe? Experimentelle Untersuchungen in vitro und in vivo. Z Kardiol 89 390 -397, 2000]. Eine über längere Zeit wirksame Arzneimittelexposition vorher erweiterter Gefäßabschnitte wurde durch langsame Freigabe von Wirkstoffen aus beschichteten Stents erreicht. Mit all diesen Verfahren bleibt es schwierig, ausreichende Wirkstoffkonzentrationen über ausreichende Zeit in den behandlungsbedürftigen Gefäßabschnitten zu erreichen. Hydrophile Wirkstoffe werden aus Geweben rasch ausgewaschen [Baumbach,A., C. Herdeg, M. Kluge, M. Oberhoff, M. Lerch, K.K. Haase, C. Wolter, S. Schröder, K.R. Karsch: Local drug delivery: Impact of pressure, substance characteristics, and stenting on drug transfer into the arterial wall. Cathet Cardiovasc Intervent 47 102 - 106, 1999]. Eine wiederholte Verabreichung ist wegen des invasiven Zugangs durch Katheter nicht möglich. Lipophile Wirkstoffe sind in gefäßverträglichen, wässrigen Medien nicht ausreichend löslich oder werden in Form von Mizellen oder Liposomen in Lösung gehalten; die Mizellen oder Liposomen werden jedoch nur langsam in das Gewebe aufgenommen. Die Verabreichung durch Spezialkatheter, die den Blutstrom für einige Zeit unterbrechen oder die Wirkstofflösung mit hohem Druck in die Gefäßwand pressen, führt zuerst einmal zu zusätzlichen Gewebeschäden, die eine Verstärkung der reaktiven Hyperplasie bewirken. Beschichtete, Arzneistoff - freisetzende Stents sind schwierig in reproduzierbarer Qualität herzustellen, enthalten wegen ihrer geringen Masse und filigranen Form nur ganz geringe Wirkstoffmengen und sind nicht geeignet, die für die Restenose wichtigen Gefäßabschnitte einige Millimeter proximal und distal des Stents zu behandeln. Wurde bereits früher ein Stent implantiert, in dessen Lumen eine Verengung auftritt, lässt sich diese durch Dilatation eines Ballonkatheters beseitigen. Die Implantation eines 2. Stents in das Lumen des 1. Stents zur Verhinderung der Gefäßwandhyperplasie als Folge der Dilatation ist jedoch unerwünscht, so dass für diesen Fall kein wirksames Verfahren zur Prophylaxe der Restenose zur Verfügung steht. Das Gleiche gilt, wenn es nach der Angioplastie keine Indikation für die Implantation eines Stents gibt oder hyperplastische Gefäßprozesse ohne deutliche Einengung des Gefäßlumens vorliegen, so dass weder eine Gefäßerweiterung noch eine Stentimplantation notwendig sind. Einige dieser Gefäßwandveränderungen können zu plötzlichen, meist thrombotisch bedingten Verschlüssen führen. Auch hier ist ein von der Stentimplantation unabhängiges Verfahren zur Hemmung der pathologischen Gefäßwandveränderungen wünschenswert.

An Wirkstoffen wurden bisher mit einigem Erfolg in experimentellen Versuchsanordnungen Heparin- und Hirudinderivate, Prostacycline, Kortikoide, Rapamycin, Kolchizin und Paclitaxel eingesetzt.
In den meisten Fällen wurden die Wirkstoffe auf Stents aufgebracht; soweit Lösungen verwendet wurden, waren dies wässrige Lösungen oder im Falle des sehr schlecht wasserlöslichen Paclitaxel (4,10β-Diacetoxy-13α-((2R,3S)-3-benzamido-2-hydroxy-3-phenylpropionyloxy)-2α-benzoyloxy-5β,20-epoxy-1,7β-dihydroxy-11-taxen-9-on) wässrige Lösungen mit Ethanol- oder Cremophor-Zusatz. Mit Cremophor [Poly(oxyethylen)-35-Rizinusöl] kommt es zur Bildung von Mizellen, die bei Verwendung von Ethanol weitgehend vermieden werden.

Zur direkten Injektion in tumorversorgende Blutgefäße sind Suspensionen oder Emulsionen mit relativ großen Partikeln in wässrigen Zytostatikalösungen ohne oder mit Kontrastmittelzusatz beschrieben worden. Diese Zubereitungen dienen dem Verschluß der Tumorgefäße bei gleichzeitiger zytostatischer Therapie. Das Verschließen der Gefäße ist jedoch dem Ziel der vorliegenden Erfindung gerade entgegengerichtet.

Der Erfindung liegt die Aufgabe zugrunde, Mittel zur örtlich begrenzten Behandlung potentiell hyperproliferativen Gewebes zur Verfügung zu stellen, welche einfach handhabbar und für den Patienten unschädlich sind.

Ausgehend vom Stand der Technik wird die Aufgabe erfindungsgemäß durch eine Zubereitung, enthaltend mindestens einen antihyperplastischen Wirkstoff mit einem Verteilungskoeffizienten zwischen Butanol und Wasser von ≥ 0.5 wobei der antihyperplastische Wirkstoff ein Cytostatikum, ein Kortikoid, ein Antibiotikum, ein Mittel zur Hemmung der Zellproliferation oder ein Immunsuppressivum ist, die in ein Mittel zur Verbesserung der bildlichen Darstellung der Arterien/Venen eingebracht oder auf einen Katheter aufgetragen wird, gelöst.

Das Prinzip der Erfindung beruht auf der überraschenden Beobachtung, dass Wirkstoffe aus hinreichend konzentrierten Lösungen, Gelen oder anderen Matrices rasch und in ausreichender Menge in die Gefäßwand aufgenommen werden, sofern sie nicht durch Lösungsvermittler in nach außen hydrophile Mizellen eingeschlossen sind. Wenn die Wirkstoffe lipophil sind (Verteilungskoeffizient Butanol : wässriger Puffer pH 7 ≥0.5, bevorzugt ≥1 und besonders bevorzugt ≥5 bzw. Octanol : wässriger Puffer pH 7 ≥ 1, bevorzugt ≥ 10, besonders bevorzugt ≥ 50) oder bzw. und reversibel (zu >10%, bevorzugt zu > 50%, besonders bevorzugt zu >80%) und/oder irreversibel an Zellbestandteile binden (z. B. Paclitaxel, Probucol (4,4'-(Isopropylidenbisthio)bis(2,6-di-tert-butylphenol), Porphyrinderivate), ist die Verweildauer in dem betroffenen Blutgefäß bei Verabreichung während der Gefäßerweiterung und ggf. Stentimplantation für den therapeutischen Effekt ausreichend. Durch Verhinderung oder Verminderung der initialen reaktiven Hyperplasie als Folge der Gefäßverletzung wird eine zu starke Verdickung der Gefäßwand über viele Monate verhindert. Überraschenderweise hat sich für die erfindungsgemäßen Zubereitungen eine längerfristige Exposition des zu behandelnden Gewebes oder eine direkte Infiltration unter zusätzlicher Verletzung der Gefäßwand als nicht notwendig erwiesen.

Im Zuge der Angioplastie und Stentimplantation werden mehrfach wiederholt Kontrastmittel selektiv in die betreffenden Gefäße injiziert, um Lage, Grad und Form der Stenose zu bestimmen, die genaue Position des Dilatationskatheters festzulegen, den Erfolg der Dilatation zu beurteilen und ggf. einen Stent geeigneter Stärke und Länge zu implantieren. Durch Zusatz der Wirkstoffe oder ihrer für diesen Zweck geeigneten Zubereitungen zu den für diagnostische Zwecke verwendeten Kontrastmitteln kommt es bei jeder Kontrastmittelinjektion ohne zusätzlichen Aufwand und ohne Beschädigung der Gefäße zu einem Wirkstoffübergang in die Gefäßwand. Dabei wird der gesamte für diagnostische Zwecke dargestellte Gefäßabschnitt behandelt, einschließlich des Bereichs deutlich vor der Stenose bis hin zu dem Gefäßabschnitt distal von der Stenose. Das hat den wesentlichen Vorteil, daß kritische Zonen vor und nach der dilatierten Stenose und ggf. Stentimplantation nicht von der Behandlung ausgeschlossen werden.

Ist die Injektion von Kontrastmitteln unnötig oder unerwünscht, können Lösungen lipophiler Wirkstoffe in anderen wässrigen Trägern ohne den Zusatz von mizellbildenden Substanzen ebenfalls eingesetzt werden. Eine Vorausetzung ist, dass diese Lösungen eine höhere Wirkstoffkonzentration enthalten als sie der Sättigungskonzentration in dem rein wässrigen Medium entspricht. Dies kann durch Zusatz z. B. von nicht und nur wenig mizellbildenden organischen Lösungsmitteln wie Ethanol und DMSO erreicht werden oder/und durch Lösung der Wirkstoffe unter Bedingungen, die der Lagerung und Verabreichung nicht zuträglich sind (z. B. Erhitzung, Mischung mit konzentrierten Wirkstofflösungen in organischen Lösungsmitteln) zur Bildung ausreichend stabiler übersättigter Lösungen.

In einigen Fällen kommt es zu einer überraschenden Verbesserung der Löslichkeit oder der Stabilität übersättigter Lösungen der wenig wasserlöslichen, lipophilen Wirkstoffe in den Kontrastmittellösungen. Ein ebenfalls überraschender Effekt ist die durch Kontrastmittel bedingte verbesserte Adhäsion und Absorption von Wirkstoffen an bzw. in die Gefäßwände sowie die gute lokale Verträglichkeit einiger systemisch extrem toxischer Substanzen in empfindlichen Gefäßgebieten.

Auch im Falle einer Inkompatibilität von Wirkstoff und Kontrastmittel oder im Falle einer ungenügenden Löslichkeit des Wirkstoffs im Kontrastmittel kann die Wirkstofflösung auch direkt durch den diagnostischen Katheter in das betreffende Gefäß infundiert oder injiziert werden. Dabei werden bevorzugt ähnliche Volumina verwendet wie sie für die Gefäßdarstellung mit Kontrastmitteln durch Katheter gebräuchlich sind [Elke M: Kontrastmittel in der radiologischen Diagnostik, S. 113-119, 3. Auflage, Georg Thieme Verlag Stuttgart New York, 1992].

Als Kontrastmittel werden gefäßverträgliche Lösungen, Suspensionen oder Emulsionen bezeichnet, die im Röntgenbild, in der Ultraschalluntersuchung, in der optischen Bildgebung oder in der Magnetresonanzdarstellung zur verbesserten Darstellung der Blutgefäße oder des Blutflusses eingesetzt werden können. Bei diesen Kontrastmitteln handelt es sich z. B. um Visipaque 320 (Iodixanol), Ultravist 370 Iopromid), Omnipaque 350 (Iohexol) oder Solutrast 370 (Iopamidol) bzw. Magnevist (Gadolinium-DPTA) oder Gadovist 1M (Gd-DO3A-butrol).

Als Wirkstoffe kommen alle zur Hemmung von Zellwachstum, Zellvermehrung und der Bildung hyperplastischer Wucherungen geeigneten Substanzen in Frage, vorausgesetzt sie erfüllen die vorn definierten Kriterien im Hinblick auf Lipophilie und/ oder Bindung an Bestandteile des Gewebes. Soweit bestimmte Wirkstoffe nicht ausreichend lipophil oder bindefähig sind, können auch deren pharmakologisch aktive Derivate oder Vorstufen der pharmakologisch aktiven Substanzen verwendet werden, die den eigentlichen Wirkstoff erst im Gewebe freisetzen. Bevorzugt sind Zytostatika aus der Gruppe der Taxoide, wie z. B. Paclitaxel und Docetaxel ((2R,3S)-N-(*tert*-Butoxycarbonyl) -2-hydroxy-3-phenyl-β-alanin- (4-acetoxy-2α-benzoyloxy-5β,20-epoxy-1,7β,10β-trihydroxy-9-oxo-11-taxen-13α-yl-ester)), oder die Epothilone als Beispiele für besonders lipophile Substanzen. Diese sind so lipophil und in Wasser unlöslich, daß auch hydrophilere Derivate, wie z.B. von Nicollaou KC, Riemer C, Kerr MA, Rideout D, Wrasidlo W. Design, synthesis and biological activity of protaxols. Nature, 1993; 364: 464-466 oder im USP 457,674, Novel Taxoids beschrieben bevorzugt werden, soweit das Molekulargewicht nicht ca. 10 kD überschreitet.
Andere nützliche Wirkstoffe gehören zu den Kortikoiden, den Mitosehemmern wie Kolchizin, den Antibiotika wie A-zithromyzin oder Roxithromyzin (Gupta et al. 1998), sowie Heparin- und Hirudinderivate. Ferner zählen auch Immunsuppressiva wie Rapamycin zu den einzusetzenden Wirkstoffen.

Beispiele für lipophile Derivate von ansonsten hydrophilen Zytostatika finden sich bei Brunner H, Schellerer K-M; Treittinger B. Synthesis and in vitro testing of hematoporphyrin type ligands in platinum(II) complexes as potent cytostatic and phototoxic antitumor agents. Inorganica Chimica Acta, 1997; 264: 67-79 in Form von Konjugaten von Platinkomplexen mit Porphyrinen.

Die erfindungsgemäßen Zubereitungen, welche als Wirkstoff ein Zytostatikum enthalten, eignen sich auch besonders zur Behandlung von Tumorerkrankungen. Vorteilhaft ist in diesem Fall, dass es sich um eine örtlich begrenzte Behandlung handelt, die die Belastung des Patienten auf ein Minimum reduziert.

Neben den lipophilen Substanzen sind auch Wirkstoffe oder an Träger gebundene Wirkstoffe mit spezifischer Affinität zu den Gefäßwänden und insbesondere zu pathologisch veränderten Gefäßwänden geeignet. Als spezifisch gefäßwandaffin werden Substanzen bezeichnet, die vom Blutstrom nicht in wenigen Minuten von den Gefäßwänden abgewaschen werden. So ist bekannt, daß sich Magnetite nach intravenöser Gabe in geringer Konzentration in arteriosklerotisch veränderten Gefäßwänden ablagern (Schmitz SA et al. Superparamagnetic iron oxide - enhanced MRI of atherosclerotic plaques in Watanabe hereditable hyperlipidemic rabbits. Invest Radiol, 2000; 35: 460-471). Allerdings ist überraschend, daß die Magnetite bei kurzeitiger Durchströmung der mittels Ballon dilatierten Gefäße für eine Therapie ausreichende Konzentrationen im Gewebe erlangen. Um die Magnetite therapeutisch nutzbar zu machen, müssen diese mit Arzneistoffen beschichtet werden wie es beispielsweise von Lübbe AS, Bergemann C, Huhnt W, Fricke T, Riess H, Brock JW, Huhn D. Preclinical Experiences with magnetic drug targeting: Tolerance and efficacy. Cancer Research, 1996; 56: 4694 - 4701) beschrieben ist.

Die Wirkstoffe werden soweit möglich in den unverdünnten Kontrastmitteln gelöst. Sie können auch als eigenständige Lösung zubereitet werden, die vor Gebrauch mit einer Kontrastmittellösung verdünnt wird. Dabei sollte das Mischungsverhältnis von Wirkstofflösung : Kontrastmittellösung nicht größer als 2:1, bevorzugt < 1:1 und besonders bevorzugt < 0.2: 1 sein. Der Wirkstoff sollte in einem gut verträglichen wässrigen oder mit Wasser mischbaren Medium gelöst werden. Zulässig sind auch organische Lösungsmittel mit guter Verträglichkeit (zumindest nach Verdünnung mit der Kontrastmittellösung oder einem anderen wässrigen Medium) wie z.B. Ethanol, DMSO, DMF etc. Meist wird jedoch ein möglichst hoher Anteil Wasser (> 90 Volumen %, bevorzugt > 95 Volumen %, besonders bevorzugt ≥ 99 Volumen %) bei der fertigen Injektionslösung angestrebt.

Der Konzentrationsbereich der einzelnen Wirkstoffe richtet sich nach deren Löslichkeit in physiologisch verträglichen Lösungsmitteln, ohne daß auf Mizellbildner wie Cremophor zurückgegriffen werden muß, sowie nach der Wirksamkeit und Verträglichkeit der Wirkstoffe. Die Obergrenze der Konzentration wird stets durch das zu verabreichende Volumen (z.B. bei mehrfacher Injektion in Koronararterien 100 - 200 ml) und die maximal systemisch verträgliche Dosis (bei Paclitaxel z.B. ca. 100 mg/ m² Körperoberfläche) bestimmt. Bevorzugt und wegen der lokalen Verabreichung und Wirkung auch ausreichend wirksam sind Dosierungen, die 1/10 oder weniger der genannten systemisch verträglichen Maximaldosis entsprechen.

Den Zubereitungen können weitere wirksame Substanzen wie Gerinnungshemmer, Plättchenaggregationshemmer, Enzymhemmer, Komplexbildner für Calciumionen etc. zugesetzt werden. Diese müssen nicht den genannten Kriterien für Lipophilie, Bindung an Gewebebestandteile oder Molekulargewicht entsprechen, da die Wirkung auch eine akute, intravasale sein kann; für die Konzentration und Dosierung gilt das im vorhergehenden Absatz Gesagte, da wiederum die lokale Wirkung in dem direkt durchströmten Gefäßabschnitt im Vordergrund steht.

Eine weitere Möglichkeit der Verabreichung antiproliferativer Wirkstoffe bietet ein zur Gefäßerweiterung eingesetzter Katheder mit einem aufweitbaren Ballon, der seinerseits die Blutgefäßaufweitung bewirkt. Der Ballon kann mit dem Wirkstoff beschichtet werden. Der Ballon wird dann bei der Gefäßaufweitung gegen die Gefäßwand gepresst. Der Wirkstoff erhält dabei die Gelegenheit, in die Gefäßwand überzutreten. Wird der Ballon zum Aufdehnen eines Stents verwendet, kann auch der zwischen dem Stent und dem Ballon befindliche Wirkstoff freigesetzt werden, da es zu Verschiebungen der Metallstreben des Stents gegenüber der Ballonoberfläche kommt. Auch bei diesen Varianten der Wirkstoffverabreichung wird gegenüber dem ursprünglichen Verfahren der Gefäßerweiterung und ggf. Stentimplantation kein zusätzlicher Arbeitsschritt für den Arzt erforderlich.

Sollen die Wirkstoffe auf den für die Gefäßaufweitung genutzten Teil des Katheders aufgebracht werden, sind folgende Verfahren möglich: Lösen des oder der Wirkstoffe in einem Lösungsmittel, das den Katheder nicht angreift, Tauchen des betreffenden Teils des Katheders in die Lösung, Entnahme des Katheders aus der Lösung und Trocknen. Der Wirkstofflösung können intravasal verträgliche matrix- oder gelbildende Hilfsstoffe zugesetzt werden, wie z. T. Lipide oder in der Pharmazie gebräuchliche Polymere. Es kann auch eine Beschichtung in mehreren Arbeitsschritten erfolgen, wobei wirkstoffhaltige und wirkstofffreie Schichten abwechseln können. Die Lösungsmittel für die jeweiligen Schichten sind so zu wählen, dass die nachfolgende Beschichtung die vorhergehende nicht wieder ablöst.

Die nachfolgenden Beispiele erläutern die Erfindung.

### Beispiele

### Beispiel 1a: Lösung für die einmalige direkte Verabreichung in die Arterien

80 mg 7-(2",3" - Dihydroxypropyl oxycarbonyl)-paclitaxel werden in 5 ml Dimethylsulfoxid gelöst und mit 5 ml 5%iger Glucoselösung verdünnt. Die Lösung oder ein Teil davon wird langsam in die vorab dilatierte Arterien infundiert.

### Beispiel 1b: Röntgenkontrastmittel mit Zusatz zur Hemmung der Intimahyperplasie

Ein Teil der unter 1a beschriebenen Lösung wird zu 99 Teilen dem handelsüblichen Röntgenkontrastmittel Visipaque 320 zugesetzt und sofort gut vermischt. Die Lösung kann in gebräuchlicher Weise für die Angiographie während und nach der Gefäßdilatation eingesetzt werden.

### Beispiel 2a: Lösung als Zusatz für Kontrastmittel

200 mg 7-(2",3" - Dihydroxypropyl oxycarbonyl)-paclitaxel werden in 10 ml absolutem Ethanol gelöst (= Lösung A); von dieser Lösung können 0.35 ml zu 100 ml Kontrastmittel zugesetzt werden.

### Beispiel 2b: Röntgenkontrastmittel zur Restenoseprophylaxe

100 ml Ultravist 370 (Schering AG, Berlin; Wirkstoff Iopromide entsprechend 370 mg Jod/ ml) enthaltend 0.35 Volumen% Ethanol und 7 mg 7-(2",3"- Dihydroxypropyl oxycarbonyl)-paclitaxel. Die Herstellung der Lösung erfolgt indem das 7-(2",3" - Dihydroxypropyloxycarbonyl)-paclitaxel zunächst in Ethanol gelöst und dann dem Kontrastmittel unter stetigem Rühren zugesetzt wird.

### Beispiel 2c: Röntgenkontrastmittel zur Restenoseprophylaxe

Zubereitung nach Beispiel 2 b mit Zusatz von 10 IE niedermolekularem Heparin

### Beispiel 2d: Restenoseinhibierende Perfusionslösung

3.5 ml der in Beispiel 2a beschriebenen Lösung A werden mit 46.5 ml Ethanol versetzt und zu 1000 ml warmer (~50 °C) 5%iger Glucoselösung oder isotoner Elektrolytlösung unter raschem Schütteln zugesetzt. Die Lösung wird wie ein Röntgenkontrastmittel durch einen Katheter in die zu behandelnden Gefäße infundiert; die Infusionsgeschwindigkeit kann gegenüber der Kontrastmittelverabreichung reduziert werden.

### Beispiel 3a: Röntgenkontrastmittel zur Hemmung der Intimahyperplasie

100 ml Ultravist 370 (s. Beispiel 2b) versetzt mit 0.4 Volumen% Ethanol und 14,4 mg 7-(2",3"- Dihydroxypropyl oxycarbonyl)-paclitaxel. Die Herstellung erfolgt wie bei Beispiel 2b.

### Beispiel 4a: Röntgenkontrastmittel zur Hemmung von Zellwachstum

100 ml Solutrast 370 (Byk-Gulden, Konstanz; Wirkstoff Iopamidol entsprechend 370 mg Jod/ ml) enthaltend 1.0 Volumen% Ethanol und 8.2 mg Paclitaxel/ ml. Zur Herstellung der Zubereitung wird Paclitaxel zunächst unter leichtem Erwärmen in absolutem Ethanol gelöst, nach dem Lösen rasch unter starkem Rühren dem Kontrastmittel zugesetzt.

### Beispiel 4b: Röntgenkontrastmittel zur Hemmung der Intimahyperplasie

Zubereitung nach Beispiel 4a unter Zusatz von 5 IE Heparin und 5 mmol/ Liter Zitratpuffer pH 7.0

### Beispiel 5a: Lösung als Zusatz zu Kontrastmitteln oder Infusionslösungen

20 mg (±)-trans-1,2-Diaminocyclohexan{7,12-bis[1-(1,4,7,10,13,16-hexaoxaheptadecyl)-ethyl]-3,8,13,17-tetramethylporphyrin-2, 18-dipropionato}platin (II) werden in 10 ml Dimethylsulfoxid gelöst (=Lösung B)

### Beispiel 5b: Röntgenkontrastmittel mit Zusatz zur Hemmung der Zellvermehrung

1 ml der Lösung B wird zu 100 ml Ultravist 370 (s. Beispiel 2b) unter raschem Rühren zugesetzt. Die Lösung ist zur Infusion in Arterien oder Injektion in lebendes oder abgestorbenes Gewebe oder in Körperhöhlen geeignet. Sie bewirkt bei ausgezeichneter Kontrolle ihrer initialen Verteilung einen längere Zeit anhaltenden zytostatischen Effekt.

Beispiel 5c: Kontrastmittel für die Magnetresonanztomographie mit Zusatz zur Hemmung der Zellvermehrung 1 ml der Lösung B wird zu 10 ml 50 mmolarer Gadolinium DTPA (= Gadopentetat) Lösung zugesetzt. Eine 50 mmolare GadoliniumDTPA-Lösung wird aus dem Handelspräparat Magnevist (Schering AG, Berlin) durch 10fache Verdünnung hergestellt. Die Lösung kann z.B. in vitale Tumore oder in Tumore nach deren Abtötung durch Ethanol, Hitze- oder Kältebehandlung infiltriert werden. Die Verteilung der Lösung ist in der Magnetresonanztomographie gut sichtbar. Die Lösung selbst unterstützt die restlose Abtötung des Tumors im unmittelbar infiltrierten Bereich und in der nahen Umgebung.

### Beispiel 6: Wirksamkeit der Zubereitung nach Beispiel 2b in vivo

Bei insgesamt 8 Schweinen wurden in Narkose je 2 Koronararterien überdehnt und Stents (feine stark perforierte Metallröhren) implantiert. Darauf reagieren die Arterien mit Wandverdickung, die zu einer Einengung des ursprünglichen Arterienlumens führt. Bei 4 Schweinen wurde zur Darstellung der Arterien und Kontrolle der Stentimplantation normales Röntgenkontrastmittel (Ultravist 370) verwendet, bei 4 Schweinen wurde die Zubereitung nach Beispiel 2b eingesetzt. Unmittelbar nach der Behandlung waren die Gefäße beider Versuchsgruppen mit 3.4±0.2 mm und 3.5±0.2 mm offenem Durchmesser praktisch gleich weit. 4 Wochen nach der Behandlung hatte sich der offene Durchmesser der Tiere, die das normale Kontrastmittel erhielten, um 1.9 ± 0.8 mm verengt, das der Tiere, die mit der Lösung nach Beispiel 2b behandelt wurden aber nur um 0.9 ± 0.6 mm. Der Unterschied ist mit p = 0.01 statistisch signifikant. Die unverdünnte Lösung nach Beispiel 2b wurde trotz des Zusatzes eines relativ toxischen Zytostatikums in hoher Konzentration nach Injektion in die Koronararterien bei gleichzeitiger EKG- und Blutdruckkontrolle ohne Nebenwirkungen vertragen.

### Beispiel 7a: Beschichtung eines Katheters

Ein für die Gefäßdilatation vorgesehener Ballonkatheter wird mit dem distalen, den Ballon tragenden Bereich unter sterilen Bedingungen in die ethanolische Lösung aus Beispiel 2a (= Lösung A) eingetaucht, für ca. 5 min. in der Lösung belassen, dann entnommen und für 2 Stunden bei Raumtemperatur getrocknet. Der Ballonkatheter kann danach in gebräuchlicher Weise für die Gefäßdilatation eingesetzt werden.
Alternativ wird im Bereich des Ballons nach dem Trocknen ein Stent aufgebracht.

### Beispiel 7b:

Es wird entsprechend Beispiel 7a vorgegangen, jedoch werden der Lösung A 100 mg pharmazeutisches Rizinusöl zugesetzt.

### Beispiel 8a:

### Löslichkeit in Kontrastmittel bzw. physiologischer NaCl-Lösung

7.6 mg Paclitaxel werden in 0.5 mg Ethanol gelöst und bei Raumtemperatur zu 50 ml Ultravist-370 (enthält 768 mg I-opromid/ml, spezifisches Gewicht ca. 1.4 g/ml) zugesetzt. Nach dem Mischen ergibt sich eine klare Lösung ohne jede Trübung, die über Tage stabil ist. Mikroskopisch sind keine Partikel in der Lösung zu erkennen.

4.2 mg Paclitaxel werden in 0.5 ml Ethanol gelöst und bei Raumtemperatur zu 50 ml 0.9%iger NaCl-Lösung zugesetzt. Nach dem Mischen ist die Zubereitung sofort trüb; nach 2h sind die meisten Partikel an der Oberfläche der Lösung zu beobachten. Mikroskopisch werden große Aggregate feiner Partikel gefunden.

Wertung: Die Löslichkeit des Paclitaxel in dem Kontrastmittel ist sehr überraschend. Die Kontrastmittellösung enthält 0.7 ml Wasser/ml Lösungsgemisch, d. h. in der Kontrastmittellösung steht dem Paclitaxel weniger Lösungsmittel zur Verfügung als in der NaCl-Lösung. Dennoch löst sich Paclitaxel besser in der Kontrastmittellösung als in der NaCl-Lösung.

### Beispiel 8b:

### Magnetit als Träger für den antihyperplastischen Wirkstoff

75 mg Paclitaxel werden in 5 ml Ethanol gelöst. Die Paclitaxellösung wird zu 50 ml einer wässrigen Zubereitung eines mit abgebautem Dextran beschichteten kolloidalen Magnetit (Konzentration bezogen auf Fe^{2+/3+} 0.5 molar, z.B. SH U 555C, Prüfpräparat der Fa. Schering AG, Berlin) zugesetzt und rasch vermischt. Die Magnetitpartikel adsorbieren Paclitaxel und tragen es nach intravenöser oder intraarterieller Injektion unter anderem in arteriosklerotisch veränderte Arterienwände und Hirntumoren. Die Dosierung erfolgt entsprechend der Verwendung des Magnetits mit ca. 50 µmol bezogen auf Fe/kg Körpergewicht.

## Patentansprüche

1. Zubereitung enthaltend mindestens einen antihyperplastischen Wirkstoff mit einem Verteilungskoeffizienten zwischen Butanol und Wasser von ≥ 0,5 in kapillargängiger flüssiger Form, wobei der antihyperplastische Wirkstoff ein Cytostatikum, ein Kortikoid, ein Antibiotikum, ein Mittel zur Hemmung der Zellproliferation oder ein Immunsuppressivum ist, zusammen mit einem Mittel zur Verbesserung der bildlichen Darstellung der Arterien/Venen.

2. Zubereitung nach Anspruch 1, die auf einen Katheter aufgetragen ist.

3. Zubereitung nach Anspruch 1 oder 2, **dadurch gekennzeichnet, dass** der antihyperplastische Wirkstoff ein Taxoid ist.

4. Zubereitung nach Anspruch 1 bis 3, **dadurch gekennzeichnet, dass** der antihyperplastische Wirkstoff Paclitaxel, Docetaxel, Probucol, ein Porphyrinderivat, Kolchizin oder Epothilon ist.

5. Zubereitung nach Anspruch 1 bis 4, **dadurch gekennzeichnet, dass** das Mittel zur Verbesserung der bildlichen Darstellung der Arterien/Venen aus gefäßverträglichen Lösungen, Suspensionen oder Emulsionen besteht, die im Röntgenbild, in der Ultraschalluntersuchung, in der optischen Bildgebung oder in der Magnetresonanzdarstellung zur verbesserten Darstellung der Blutgefäße oder des Blutflusses eingesetzt werden.

6. Zubereitung nach Anspruch 1 bis 5, **dadurch gekennzeichnet, dass** das Mittel zur Verbesserung der bildlichen Darstellung der Arterien/Venen ein Röntgenkonstrastmittel ist.

7. Zubereitung nach Anspruch 1 bis 6, **dadurch gekennzeichnet, dass** das Mittel zur Verbesserung der bildlichen Darstellung der Arterien/Venen lodixanol (Visipaque), Gadolinium-DTPA (Magnevist), Gd-DO3A-butrol (Gadovist), lopromid (Ultravist), lohexol (Omnipaque) oder lopamidol (Solutrast) ist.

8. Zubereitung nach Anspruch 1 bis 7, **dadurch gekennzeichnet, dass** der antihyperplastische Wirkstoff Paclitaxel und das Mittel zur bildlichen Darstellung der ArterienNenen lopromid (Ultravist) ist.

9. Zubereitung nach Anspruch 1 bis 8, **dadurch gekennzeichnet, dass** sie zusätzlich Gerinnungshemmer, und/oder Plättchenaggregationshemmer und/oder Enzymhemmer und/oder Calciumchelatoren enthält.

10. Zubereitung nach Anspruch 1 bis 9, worin der Wirkstoff irreversibel oder reversibel zu mindestens 10 % an Gewebe bindet.

11. Zubereitung nach Anspruch 1 bis 10, worin der Wirkstoff oder die wirkstofftragende Komponente eine spezifische Affinität zu Gefäßwänden aufweist.

12. Zubereitung nach Anspruch 1 bis 11, **dadurch gekennzeichnet, dass** sie einen nicht mizellbildenden Lösungsvermittler enthält.

13. Verwendung der Zubereitung nach Anspruch 1 bis 12 zur Herstellung eines Mittels für die Prophylaxe der Restenose.

14. Verwendung der Zubereitung nach Anspruch 1 bis 12 zur Herstellung eines Mittels für die Behandlung von Tumorerkrankungen.

## Claims

1. A preparation containing at least one antihyperplastic agent with a distribution ratio between butanol and water of ≥0.5 in liquid form suitable for passing through capillaries, wherein the antihyperplastic agent is a cytostatic, a corticoid, an antibiotic, an agent inhibiting cell proliferation or an immunosuppressant together with an agent for enhancing artery/vein imaging.

2. The preparation of claim 1 which is applied to a catheter.

3. The preparation according to claim 1 or 2, wherein the antihyperplastic agent is a taxoid.

4. The preparation of any one of claims 1-3, wherein the antihyperplastic agent is paclitaxel, docetaxel, probucol, a porphyrin derivative, colchicine or epothilone.

5. The preparation according to any one of claims 1-4, wherein the agent for enhancing artery/vein imaging consists of solutions, suspensions or emulsions that the vessels can tolerate and that are used in X-raying, sonography, optical imaging, or magnetic resonance imaging for improved representation of the blood vessels or the bloodstream.

6. The preparation according to any one of claims 1-5, wherein the agent for enhancing artery/vein imaging is an X-ray contrast medium.

7. The preparation according to any one of claims 1-6, wherein the agent for enhancing artery/vein imaging is iodixanol (Visipaque), gadolinium-DTPA (Magnevist), Gd-D03A-butrol (Gadovist), iopromide (Ultravist), iohexol (Omnipaque), or iopamidol (Solutrast).

8. The preparation of any one of claims 1-7, wherein the antihyperplastic agent is paclitaxel and the agent for enhancing artery/vein imaging is iopromide (Ultravist).

9. The preparation according to any one of claims 1-8, **characterized in that** the preparation additionally contains coagulation inhibitors and/or platelet aggregation inhibitors and/or enzyme inhibitors and/or calcium chelators.

10. The preparation according to any one of claims 1-9, wherein the active agent irreversibly or reversibly binds to tissue at a minimum percentage of 10%.

11. The preparation according to any one of claims 1-10, wherein the active agent or the component carrying the active agent comprises a specific affinity to vascular walls.

12. The preparation according to any one of claims 1-11, wherein the preparation contains a solubility promoter that does not form micelles.

13. Use of the preparation according to any one of claims 1-12 for the manufacture of an agent for restenosis prevention.

14. Use of the preparation according to any one of claims 1-13 for the manufacture of an agent for treating tumor diseases.

## Revendications

1. Préparation comprenant au moins un agent actif antihyperplastique avec un coefficient de partage entre le butanol et l'eau ≥ 0,5, sous une forme apte à s'écouler dans des capillaires, dans laquelle l'agent actif antihyperplastique est un cytostatique, un corticoïde, un antibiotique, un agent d'inhibition de la prolifération cellulaire ou un immunosuppresseur, conjointement avec un produit pour améliorer la représentation graphique des artères/veines.

2. Préparation selon la revendication 1, qui est appliquée sur un cathéter.

3. Préparation selon la revendication 1 ou 2, **caractérisée en ce que** l'agent actif antihyperplastique est un taxoïde.

4. Préparation selon l'une quelconque des revendications 1 à 3, **caractérisée en ce que** l'agent actif antihyperplastique est le paclitaxel, le docetaxel, le probucol, un dérivé de porphyrine, la colchicine ou l'épothilone.

5. Préparation selon l'une quelconque des revendications 1 à 4, **caractérisée en ce que** le produit pour améliorer la représentation graphique des artères/veines consiste en solutions, suspensions ou émulsions que les vaisseaux peuvent tolérer et qui sont utilisées en radiographie aux rayons X, échographie, imagerie optique ou imagerie par résonance magnétique, pour une représentation améliorée des vaisseaux sanguins ou du flux sanguin.

6. Préparation selon l'une quelconque des revendications 1 à 5, **caractérisée en ce que** le produit pour améliorer la représentation graphique des artères/veines est un produit de contraste de radiographie aux rayons X.

7. Préparation selon l'une quelconque des revendications 1 à 6, **caractérisée en ce que** le produit pour améliorer la représentation graphique des artères/veines est l'iodixanol (Visipaque), le gadolinium-DTPA (Magnevist), le Gd-DO3A-butrol (Gradovist), l'iopromide (Ultravist), l'iohexol (Omnipaque), ou l'iopamidol (Solutrast).

8. Préparation selon l'une quelconque des revendications 1 à 7, **caractérisée en ce que** l'agent actif antihyperplastique est le Paclitaxel et le produit pour améliorer la représentation graphique des artères/veines est l'iopromide (Ultravist).

9. Préparation selon l'une quelconque des revendications 1 à 8, **caractérisée en ce qu'**elle contient, en outre, des inhibiteurs de la coagulation, et/ou des inhibiteurs de l'agrégations des plaquettes et/ou des inhibiteurs d'enzymes et/ou des agents chélatant du calcium.

10. Préparation selon l'une quelconque des revendications 1 à 9, dans laquelle l'agent actif se lie de manière irréversible ou réversible jusqu'à au moins 10 % au tissu.

11. Préparation selon l'une quelconque des revendications 1 à 10, dans laquelle l'agent actif ou le composant supportant l'agent actif présente une affinité spécifique pour les parois vasculaires.

12. Préparation selon l'une quelconque des revendications 1 à 11, **caractérisée en ce qu'**elle contient un promoteur de solubilisation qui ne forme pas de micelles.

13. Utilisation de la préparation selon l'une quelconque des revendications 1 à 12 pour la fabrication d'un produit destiné à la prophylaxie de la resténose.

14. Utilisation de la préparation selon l'une quelconque des revendications 1 à 12 pour la fabrication d'un produit destiné au traitement de maladies tumorales.
